(19) Europäisches Patentamt / European Patent Office / Office européen des brevets

(11) **EP 2 230 991 B1**

(12) **EUROPEAN PATENT SPECIFICATION**

(45) Date of publication and mention
of the grant of the patent:
**04.07.2018 Bulletin 2018/27**

(21) Application number: **08865380.3**

(22) Date of filing: **25.12.2008**

(51) Int Cl.:
**A61B 5/00** *(2006.01)* **G06F 19/00** *(2018.01)*

(86) International application number:
**PCT/IL2008/001668**

(87) International publication number:
**WO 2009/081404 (02.07.2009 Gazette 2009/27)**

(54) **SYSTEM FOR GLYCEMIC CONTROL**

SYSTEM ZUR BLUTZUCKERKONTROLLE

SYSTÈME DE CONTRÔLE GLYCÉMIQUE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **26.12.2007 US 9296 P**

(43) Date of publication of application:
**29.09.2010 Bulletin 2010/39**

(73) Proprietors:
• **Roche Diabetes Care GmbH**
**68305 Mannheim (DE)**
Designated Contracting States:
**DE**
• **F. Hoffmann-La Roche AG**
**4070 Basel (CH)**
Designated Contracting States:
**AT BE BG CH CY CZ DK EE ES FI FR GB GR HR HU IE IS IT LI LT LU LV MC MT NL NO PL PT RO SE SI SK TR**

(72) Inventors:
• **YODFAT, Ofer**
**71725 Modi'in (IL)**
• **SHAPIRA, Gali**
**34558 Haifa (IL)**

(74) Representative: **Evens, Paul Jonathan**
**Maguire Boss**
**24 East Street**
**St. Ives, Cambridgeshire PE27 5PD (GB)**

(56) References cited:
**EP-A- 0 881 495      EP-A- 1 177 802
EP-A- 1 845 465      WO-A-2004/015539
US-A- 5 139 023      US-A1- 2004 096 959**

• **BEACH K W: "A THEORECTICAL MODEL TO PREDICT THE BEHAVIOR OF GLYCOSYLATED HEMOGLOBIN LEVELS" JOURNAL OF THEORETICAL BIOLOGY, ACADEMIC PRESS, LONDON, GB, vol. 81, no. 3, 1 January 1979 (1979-01-01), pages 547-561, XP001056200 ISSN: 0022-5193**
• **ROHLFING CURT L ET AL: "Defining the relationship between plasma glucose and HbA(1c): analysis of glucose profiles and HbA(1c) in the Diabetes Control and Complications Trial" DIABETES CARE, AMERICAN DIABETES ASSOCIATION, ALEXANDRIA, VA, US, vol. 25, no. 2, 1 February 2002 (2002-02-01), pages 275-278, XP002501230 ISSN: 0149-5992**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

CROSS-REFERENCE TO RELATED APPLICATIONS

This application claims priority to provisional U.S. application Serial No. 61/009,296 entitled "DEVICE AND METHOD FOR GLYCEMIC CONTROL", and filed December 26, 2007.

FIELD OF THE INVENTION

The present disclosure relates generally to a system for assessing the glycemic control of diabetes patients. More particularly, the disclosure relates to a system (or device) that continuously and/or periodically, monitors bodily analytes and can continuously and/or periodically deliver therapeutic fluids. The disclosure also relates to a system that contains a glucose sensor and a processor for assessing the user glycemic status, and can include a portable insulin dispenser. Additionally, the disclosure relates to a system which includes an insulin dispenser and which can continuously and/or periodically monitor levels of bodily glucose and a processor to assess the user's glucose variability and hemoglobin A1C (HbA1c).

BACKGROUND

Diabetes and Glycemic Control

Diabetes mellitus is a disease of major global importance, increasing in frequency at almost epidemic rates, such that the worldwide prevalence of the disease in 2006 was 170 million people and is predicted to at least double over the next 10-15 years. Diabetes is characterized by a chronically raised blood glucose concentration (hyperglycemia), due to a relative or absolute lack of the pancreatic hormone, insulin. Within the healthy pancreas, beta cells, located in the islets of Langerhans, continuously produce and secrete insulin according to the blood glucose levels, thus maintaining near constant glucose levels in the body.

Diabetes can cause acute and chronic complications. Acute complications include hypoglycemia and ketoacidosis. Long-term complications, due to the affect on small and large blood vessels, include eye, kidney, and nerve damage and accelerated atherosclerosis, with increased rates of coronary heart disease, peripheral vascular disease and stroke.

The Diabetes Control and Complications Trial (DCCT) demonstrated that development and progression of the chronic complications of diabetes are greatly related to the degree of altered glycemia as quantified by determinations of glyco Hemoglobin (HbAlc). [DCCT Trial, N Engl J Med 1993; 329: 977-986, UKPDS Trial, Lancet 1998; 352: 837-853. BMJ 1998; 317, (7160): 703-13 and the EDIC Trial, N Engl J Med 2005; 353, (25): 2643-53].

Insulin infusion pumps

Frequent insulin administration can be done by multiple daily injections (MDI) with syringe or by continuous/periodic subcutaneous insulin injection (CSII) performed by insulin pumps. In recent years, ambulatory portable insulin infusion pumps have emerged as a superior alternative to multiple daily injections of insulin. These pumps, which deliver insulin at a continuous/periodic basal rate as well as in bolus volumes, were developed to liberate patients from repeated self-administered injections, and to enable greater flexibility in dose administration.

Several ambulatory insulin infusion devices are currently available on the market. The first generation of such devices employs disposable syringe-type reservoir and tubes. These devices have been described, for example, in U.S. Patents Nos. 3,771,694, 4,657,486 and 4,498,843. A drawback of these devices is their large size and weight, caused by their spatial configuration and the relatively large driving mechanism associated with the syringe and the piston. These relatively bulky devices have to be carried in a patient's pocket or be attached to a belt. Consequently, the fluid delivery tube becomes long, e.g., longer than 40cm, to enable needle insertion in remote locations of the body. These uncomfortable bulky devices with a long tube are disfavored by many diabetic insulin users because they disturb regular activities, such as sleeping, physical activities (e.g., swimming), etc. In addition, the long delivery tube is not suitable for use with some optional remote insertion sites such as the buttocks and the extremities.

To avoid the shortcomings associated with the necessity of using long delivery tube, a new concept, implemented as second generation devices, was proposed and developed.

Devices and systems based on the second generation concept included a housing having a bottom surface adapted for contact with the patient's skin, a reservoir disposed within the housing, and an injection needle adapted for communication with the reservoir. These skin adherable devices could be disposed of every 2-3 days, as is the case with current pump infusion sets. These devices, conforming to the second generation paradigm, are described, for example, by Schneider in US Patent No. 4,498,843, Burton in US Patent No. 5,957,895, Connelly, in US Patent No. 6,589,229, and by Flaherty in US Patents No. 6,740,059. Other configurations of skin securable (e.g., adherable) pumps are disclosed, for example, in US patents 6,723,072 and 6,485,461. In these patents, the pump is generally configured as a single piece securable (adherable) to the patient skin for the entire usage duration. The needle emerges from the bottom surface of the device and is fixed to the device housing. A disadvantage of these second-generation skin securable (e.g., adherable) devices lies in the fact that they are generally expensive, bulky and heavy.

To avoid these shortcomings, a 3rd generation

of skin securable (e.g., adherable) pump devices were proposed and developed, as described, for example, in U.S. Patent Application No. 11/397,115, published as US 2007/0106218. This pump is configured as a miniature portable programmable dispensing patch that has no tubing and that can be attached to the patient's skin. In some embodiment, this pump includes two parts, a disposable part that contains a reservoir and an outlet port, and a reusable part that contains the electronic parts, a driving mechanism, and other types of relatively expensive components and/or units. This pump device may, in some embodiments, include a remote control unit that enables data acquisition, programming, and entry of user inputs.

Insulin pump and continuous glucose monitors (CGM)

Insulin pumps can communicate with analyte sensors, such as a continuous glucose monitor (CGM), as described for example in U.S. patent 6,558,351. These discrete devices (i.e., the sensor and the pumping device) are relatively bulky, expensive devices that require two infusion sets with long tubing and two insertion sites. A new generation of a dual function device is described, for example, in U.S. Patent Applications Nos. 11/706,606 and 11/963,481, and International Patent Application No. PCT/IL07/001579, published as WO 2008/078319. That device is a single skin securable (adherable) patch employing a single subcutaneous cannula configured to perform drug infusion and analyte sensing.

Hemoglobin A1c

*Glycemic control* is a medical term referring to disease severity status in a person with diabetes mellitus, or the ability to maintain normal glucose levels and to avoid complications (neuropathy, nephropathy, retinopathy, etc.).

Glycosylated hemoglobin (HbAlc) is a form of hemoglobin used primarily to identify the patient glycemic control over prolonged periods of time (e.g., 3 months). It is formed in a non-enzymatic pathway by hemoglobin's normal exposure to high plasma levels of glucose. A high HbAlc represents poor glycemic control.

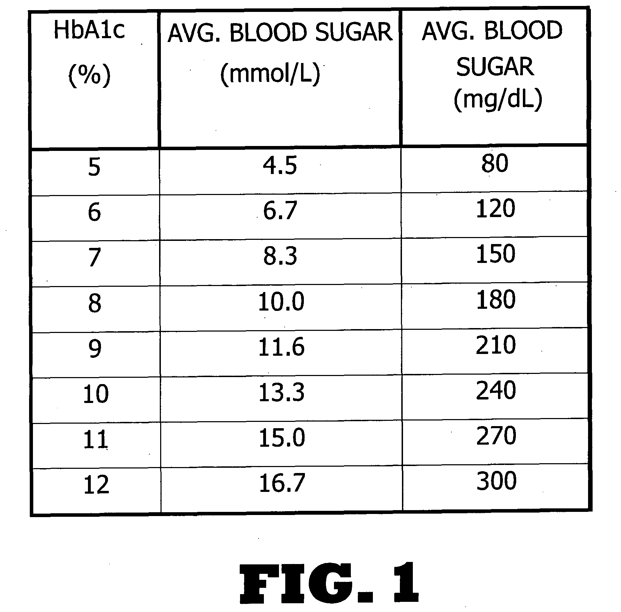

The approximate mapping between HbAlc values and average blood glucose measurements over the previous 4-12 weeks is shown in the table depicted in Figure 1 (table available from public sources).

The above-referenced table is inaccurate, partly because the level of HbAlc does not reflect the weighted mean of the preceding measured bodily glucose concentration, but rather a simple mean. BG excursions that are more recent generally contribute more than earlier events to a currently measured HbAlc level.

Currently, diabetes patients may monitor the glycemic status by periodic measurements of their HbAlc levels. A follow-up visit is usually required to obtain and discuss the results. Recently, mathematical models have been developed that exhibit the relationship between HbAlc and blood glucose. One such model is the model developed by the team of Siv M. Ostermann-Golker and Hubert W. Vesper (Journal of Diabetes and its Complications 2006, 20,285-294).

EP-A-1845465 relates to methods and systems for monitoring the effectiveness of diabetes treatment, and discloses a portable insulin dispensing system according to the precharacterizing portion of appended independent claim 1. US2004/0096959 relates to apparatus for and methods of measuring certain properties of body fluids and the concentrations of analytes therein.

SUMMARY OF THE EMBODIMENTS

In accordance with the present invention, there is provided a portable insulin dispensing system as defined in appended independent claim 1. Embodiments are defined in the appended claims which depend from independent claim 1. In some embodiments, a system that can periodically assess the HbA1c and/or glucose variability of a user by frequent bodily glucose measurements is provided.

In some embodiments, a system that can continuously monitor glucose levels configured to assess the user's HbAlc value and/or glucose variability is provided.

In some embodiments, a system/device that includes a skin adherable continuous glucose sensing patch configured to assess the user's HbAlc value and/or glucose variability is provided.

In some embodiments, an insulin dispensing and glucose sensing system configured to assess the user's HbAlc value and/or glucose variability is provided.

In some embodiments, an insulin dispensing and continuous glucose sensing system configured to assess the user's HbAlc value and/or glucose variability is provided.

In some embodiments, a system which is miniature, discreet, economical for the users and cost effective that is configured to assess the user's HbAlc value and/or glucose variability is provided.

In some embodiments, a system that includes a continuous glucose sensing patch that can be remotely controlled and that is configured to assess the user's HbAlc value and/or glucose variability is provided.

In some embodiments, a system that includes a continuous glucose sensing and insulin dispensing patch that can be remotely controlled and that is configured to assess the user's HbAlc value and/or glucose variability is provided.

In some embodiments, a system that includes a miniature skin adherable glucose sensing patch that can continuously and/or periodically dispense insulin and monitor body glucose concentration levels, and that is configured to assess the user's HbAlc value and/or glucose variability is provided.

In some embodiments, a system that includes a miniature skin adherable glucose sensing and insulin dispensing patch that can continuously/periodically dispense insulin and monitor body glucose concentration levels, and that is configured to assess the user's HbAlc value and/or glucose variability is provided.

The present disclosure describes a system that continuously monitors bodily analyte levels (e.g., glucose) and can deliver therapeutic fluid into the body (e.g., insulin).

In some embodiments, the system includes a continuous glucose monitoring unit (CGM) that can periodically assess the user's glycemic control by providing a HbAlc value that is derived from a mathematical model that exhibits the relationship between HbAlc values and bodily glucose concentrations. The user's glycemic control may be determined at any given time, based on the glycemic control during the preceding 120 days (e.g., based on BG measurements over the last 120 days, from which HbAlc values may be determined). The user's glycemic control can also be determined (e.g., computed) according to the user's glucose variability. In some embodiments, the system comprises, in addition to a CGM, an insulin dispensing unit. The CGM and insulin dispensing unit may be disposed in a single housing. In some embodiments, a remote control unit may be used that is configured to communicate with the CGM and dispensing patch unit and to enable programming of therapeutic fluid delivery, user input and data acquisition. In some embodiments, the CGM and dispensing patch unit arrangement is composed of two parts - a disposable part and reusable part. The disposable part contains a reservoir and outlet port. The reusable part contains, in some embodiments, electronics (PCB, processor, etc), a driving mechanism a metering portion, and other relatively expensive components. In some embodiments, a glucometer may be integrated into the remote control unit to enable calibration of the CGM (i.e., a calibration unit). In some embodiments, the fluid delivered by the sensing and dispensing patch unit is adjusted by a processor according to the detected glucose concentrations in a closed or semi-closed loop system.

In some embodiments, a user's HbAlc levels may be determined based on a mathematical model representing the relationship between HbAlc values and bodily glucose concentrations.

In some embodiments described herein, a system that can periodically determine the HbAlc and/or glucose variability of the user by frequent bodily glucose measurements is provided.

In some embodiments described herein, a system for continuous/periodic glucose sensing configured to assess the user's HbAlc value and/or glucose variability is provided.

In some embodiments described herein, a system that comprises a skin adherable continuous glucose sensing patch configured to assess the user's HbA1c value and/or glucose variability is provided.

In some embodiments described herein, a system that can continuously/periodically dispense insulin and monitor glucose levels and configured to assess the user's HbAlc value and/or glucose variability is provided.

In some embodiments described herein, a system which comprises a glucose sensing and insulin dispensing unit that is miniature, discreet, economical for the users and cost effective, and that is configured to assess the user's HbAlc value and/or glucose variability, is provided.

In some embodiments described herein, a system that comprises a continuous glucose sensing patch that can be remotely controlled and that is configured to assess the user's HbAlc value and/or glucose variability is provided.

In some embodiments described herein, a system that comprises a continuous glucose sensing and insulin dispensing patch that can be remotely controlled and that is configured to assess the user's HbAlc value and/or glucose variability is provided.

In some embodiments described herein, a system that comprises a miniature skin adherable glucose sensing patch that can continuously and/or periodically dispense insulin and monitor body glucose concentration levels and that is configured to assess the user's HbAlc value and/or glucose variability is provided.

In some embodiments described herein, a system that comprises a miniature skin adherable glucose sensing and insulin dispensing patch unit that can continuously dispense insulin and monitor body glucose concentration levels and that is configured to assess the user's HbAlc value and/or glucose variability is provided.

In some embodiments, the HbAlc assessment is established via processing of information obtained from frequent glucose measurements (e.g., subcutaneous continuous glucose monitoring).

In some embodiments, the HbAlc assessment can be obtained from a mathematical model exhibiting the relationship between HbAlc and interstitial fluid (ISF) glucose.

In some embodiments, the HbAlc assessment can be obtained from two mathematical models: 1) exhibiting the relationship between HbAlc and blood glucose, and 2) exhibiting the relationship between ISF and blood glucose.

In some embodiments, a mathematical model exhibiting the relationship between HbAlc and blood glucose can be applied to ISF glucose avoiding the need for a model that transforms ISF glucose to BG.

In some embodiments, the HbAlc can be assessed using one or more of conventional models, e.g., the mathematical model exhibiting the relationship between HbAlc level and blood glucose developed by the team of Siv M. Ostermann-Golker and Hubert W. Vesper (Journal of Diabetes and its Complications 2006, 20,285-294). Alternatively, simpler models may be applied, for example, the model developed by Beach (1979) which is based on simple first order reaction kinetics between glucose and hemoglobin, loss of HbAlc through erythrocyte clearance, and in which HbAlc is assumed to being a stable reaction product.

In some embodiments, the applied model requires inputting the time-weighted averaged bodily glucose values over a predefined period, e.g., 1 day. That is, the level of HbAlc does not reflect the simple mean, but rather a weighted mean of the preceding measured bodily glucose concentration. More recent BG events may thus contribute relatively more to the final HbAlc result than earlier events.

In some embodiments, 50% of HbAlc assessed value is determined by the BG levels during the preceding 35 days, 25% by the BG levels during 25 day period before that period, and the remaining 25% during the 2 month period before these periods (as indicated, for example, in Diabetes Care, 2006, 29, (2), 466-467).

In some embodiments, if a high HbAlc value is assessed, the user gets an alert from the system recommending a medical check by a health practitioner (e.g., to perform a dilated eye examination, serum creatinine, etc.), and possibly performing pump setting changes.

In some embodiments, glucose variability may be determined using a continuous glucose sensor, and may be considered alone, or in combination with the assessed HbAlc value as an indicator of glycemic control and predictor of long-term complications (e.g., retinopathy, nephropathy).

In some embodiments, glucose variability is defined by one or more of, for example, standard deviation of bodily glucose values, duration of normal, low or high readings, mean amplitude of glycemic excursions and/or glycemic lability index (as indicated, for example, in Diabetes 2004, 53, 955-962).

In some embodiments, the HbAlc value assessment procedure is implemented in a system that continuously and/or periodically monitors body glucose levels. Such a system may be a patch unit that can be adhered to the user's skin. The patch unit may comprise a reusable part and a disposable part. In some embodiments, the system comprises, in addition to the CGM patch unit, a remote control unit wherein a blood glucose monitor (e.g., glucometer) is integrated in the remote control unit for periodic calibration of the CGM patch unit.

The blood glucose monitor (e.g., glucometer) may alternatively be integrated in the reusable part of the CGM patch unit of the system.

The HbAlc value assessment procedure may be implemented in the remote control unit of the system. Alternatively, the HbAlc value assessment method could be implemented in the reusable part of the CGM patch unit of the system.

In some embodiments, the HbAlc value assessment procedure is implemented in a system that continuously monitors body glucose levels and can concomitantly deliver insulin into the body. The system may be a patch unit that can be secured (e.g., adhered) to the user's skin. The patch unit may include a reusable part and a disposable part. The insulin dispensing and glucose sensing capabilities can be combined into a semi closed loop system, where a processor-controller apparatus regulates the dispensing of basal insulin according to the sensed glucose concentration. According to some embodiments, the system may include a remote control unit to enables programming of therapeutic fluid delivery, user input and data acquisition.

The HbAlc value assessment procedure may be implemented in the remote control unit of the system. Alternatively, the procedure may be implemented in the reusable part of the CGM and insulin dispensing patch unit of the system. Alternatively, the procedure could be implemented in both the reusable part of the patch unit of the system and the remote control unit of the system.

In one aspect, a glucose monitoring system is disclosed. The system includes a glucose sensor to periodically perform a plurality of glucose measurements in interstitial fluid, and a processor to determine one or more HbAlc values representative of a patient's glycosylated hemoglobin levels based on the periodic glucose measurements.

Embodiments of the system may include one or more of the following features.

The glucose sensor may be coupled to a fluid dispensing pump.

In another aspect, a portable insulin dispensing system is disclosed. The system includes a portable insulin dispensing pump to deliver insulin to a patient, a glucose sensor to periodically perform a plurality of glucose measurements corresponding to glucose concentration in the blood, and a processor to determine one or more HbAlc values representative of a patient's glycosylated hemoglobin levels based on the periodic glucose measurements.

Embodiments of the system may include any of the features described in relation to the first system above, as well as any of the following features.

The processor is provided in any one or more of, for example, the portable insulin dispensing system, a handheld remote control unit for the insulin dispensing pump and/or a handheld blood glucose monitor.

The glucose sensor may be configured to periodically perform a plurality of glucose measurements in interstitial fluid.

The glucose sensor may be coupled to the dispensing pump.

The processor may be further configured to compute one or more values representative of the patient's glucose variability levels.

At least part of the pump may be securable to the patient's skin.

The pump may include the glucose sensor.

The pump may include a disposable part and a reusable part.

The one or more HbAlc values are determined based on a mathematical model relating the one or more HbAlc values to glucose concentrations determined from the glucose measurements. The mathematical model includes a first order reaction kinetics model representative of a first order reaction kinetics between glucose and hemoglobin and loss of HbAlc through erythrocyte clearance in which HbAlc is estimated to be a stable reaction product.

The one or more HbA1c values may be computed based on a time-weighted averaged computation of the glucose measurements.

A method for monitoring glycemic control in a patient is disclosed. The method includes providing a skin securable housing including a glucose sensor, periodically performing a plurality of glucose measurements in interstitial fluid, and determining one or more HbAlc values representative of a patient's glycosylated hemoglobin levels based on the periodic glucose measurements.

The method may include one or more of the features described in relation to the above systems, as well as any of the following features.

Determining the one or more HbAlc values may include determining the one or more HbAlc values using a processor disposed in any one or more of, for example, the skin securable housing, a handheld remote control unit for an insulin dispensing pump and/or a handheld blood glucose monitor.

Providing the glucose sensor may include providing a glucose sensor coupled to a fluid dispensing pump.

Another method for monitoring glycemic control in a patient is disclosed. The method includes delivering insulin into the body of a patient using an insulin dispensing device, performing periodically a plurality of glucose measurements, the glucose measurements performed using a sensor coupled to the dispensing device, and determining one or more HbAlc values representative of a patient's glycosylated hemoglobin levels based on the periodic glucose measurements.

The method may include one or more of the features described in relation to the above systems and method, as well as any of the following features.

The method may further include providing a processor in any one or more of, for example, the dispensing device, a handheld remote control unit for the insulin dispensing device and/or a handheld blood glucose monitor, the processor configured to determine the one or more HbAlc values.

Performing periodically the plurality of glucose measurements may include performing a plurality of glucose measurements in interstitial fluid.

The method may further include determining one or more values representative of the patient's glucose variability levels.

Determining the one or more HbAlc values may include computing the one or more HbAlc values based on a mathematical model relating the one or more HbAlc values to the glucose measurements. The mathematical model includes a first order reaction kinetics model representative of a first order reaction kinetics between glucose and hemoglobin and loss of HbAlc through erythrocyte clearance in which HbAlc is estimated to be a stable reaction product.

Computing the one or more HbAlc values may include computing the one or more HbAlc values based on a time-weighted averaged computation of the glucose measurements.

Performing periodically the plurality of glucose measurements may include performing periodically glucose concentration level of the patient using a continuous glucose monitoring unit (CGM).

The method may further include periodically calibrating, using a glucometer, one or more of, for example, the sensor and/or a continuous glucose monitoring unit (CGM) performing the glucose measurements.

## BRIEF DESCRIPTION OF THE DRAWINGS

FIG. 1 is a table of HbA1c and blood glucose averages.

FIGS. 2a-c are schematic diagrams of an exemplary continuous glucose monitoring (CGM) device and a remote control unit configured to perform HbAlc computations.

FIGS. 3a-d are schematic diagrams of an exemplary system that includes a CGM with an insulin dispensing unit and a remote control unit configured to perform HbAlc computations.

FIGS. 4a-b are schematic diagrams of exemplary insulin infusion systems containing two embodiments of continuous subcutaneous glucose monitors to provide blood glucose readings (BG) for the HbAlc computation.

FIG. 5 are equations representative of an exemplary mathematical model developed by the team of Siv M. Ostermann-Golker and Hubert W. Vesper.

FIG. 6 is a block diagram of an exemplary procedure to determine HbAlc levels.

FIG. 7 is an anatomical/physiological schematic depicting an exemplary data acquisition model to determine the glucose concentration levels in the ISF.

FIG. 8 is a diagram of an exemplary embodiment of an HbAlc computation implementation using a remote control unit and PC.

Like reference numbers and designations in the various drawings indicate like elements.

## DETAILED DESCRIPTION

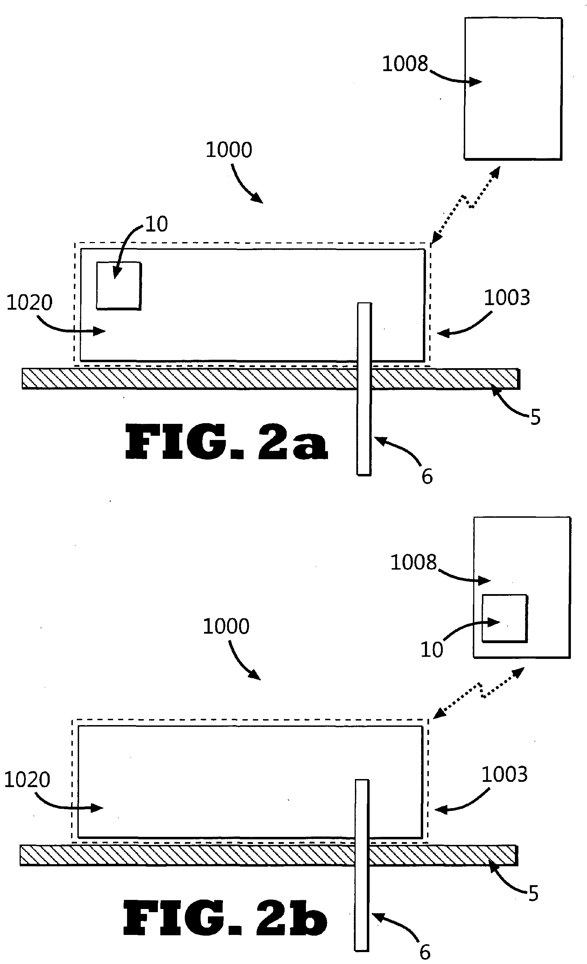

Referring to Figures 2a-c, schematic diagrams of exemplary embodiments of a system 1000 for continuous or periodic glucose monitoring (e.g., CGM) are shown. The system 1000 includes, without any limitations, a CGM unit 1020 and optionally a remote control unit 1008. The CGM unit is connected to a cannula 6 that penetrates the patient's skin 5 and resides in the subcutaneous tissue for sensing the glucose concentration in the interstitial fluid (ISF). Other types of probes (e.g., non-tubular probes) to sense and/or measure glucose concentrations in the ISF may be used.

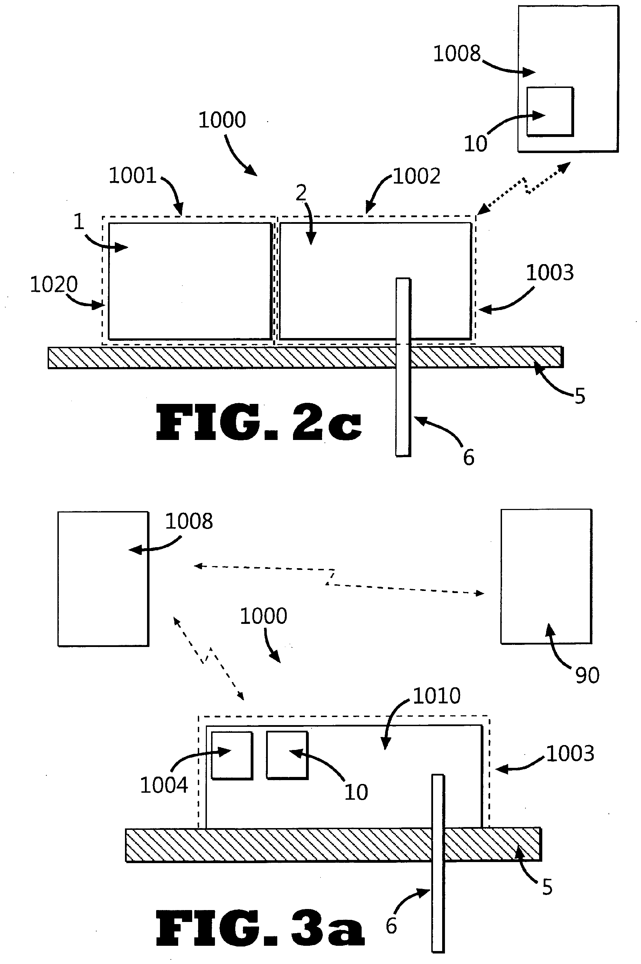

The CGM unit 1020 may be contained in a single housing 1003, as shown, for example, in Figures 2a-b, or in two housings 1001, 1002, comprising a reusable part 1 and a disposable part 2 respectively, as shown in Figure 2c. Data acquisition may be performed by the remote control unit 1008. The CGM unit 1020 may be configured as a patch that can be directly attached to the patient's skin 5 by adhesives (not shown) or, in some embodiments, it may be connected to a dedicated cradle unit (not shown) that is adhered to the patient skin 5 and enables the CGM patch unit 1020 disconnection from and reconnection to the body.

A module to implement HbAlc computation (also referred to as the HbAlc assessment feature) 10 can be located in the CGM unit 1020 (as shown in Figure 2a), or in the remote control unit 1008 (as shown, for example, in Figures 2b-c).

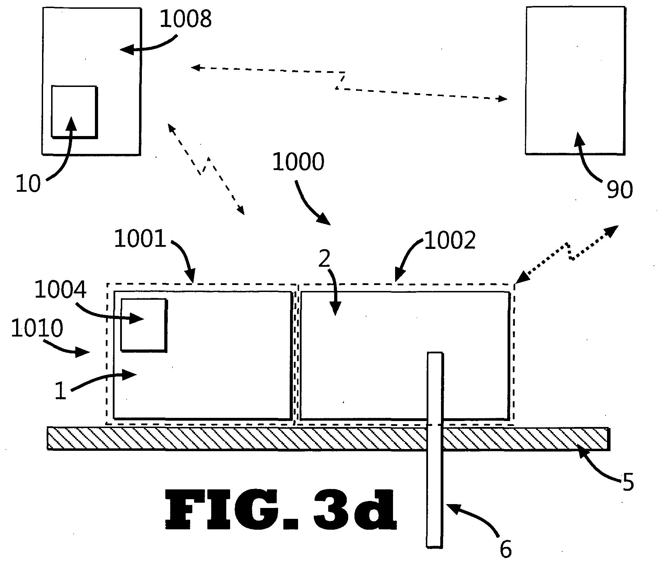

Referring to Figures 3a-d, schematic diagrams of exemplary embodiments of a system 1000 configured to perform continuous sensing of ISF glucose levels (CGM) and to dispense therapeutic fluids (e.g., insulin) to the body are shown. The system 1000 includes a sensing and dispensing unit 1010, a remote control unit 1008, and a blood glucose (BG) monitor 90 to enable calibration of the CGM. In some embodiments, the unit 1010 may not include a CGM (for ISF glucose measurements), and thus, under those circumstances, BG measurements performed by a blood glucose monitor 90 may be used for HbAlc determination. The sensing and dispensing unit is connected to a cannula 6 that delivers the drug through the skin 5 into the body. The sensing and dispensing unit can be contained in a single housing 1003, as shown, for example, in Figures 3a-c, or in two housings 1001, 1002, comprising a reusable part 1 and a disposable part 2, respectively, as shown, for example, in Figure 3d. Programming functionality (e.g., to specify infusion/flow profiles) and data acquisition may be performed by a remote control unit 1008, or may alternatively be performed directly by operating buttons 1004 located on the dispensing unit housing. In some embodiments, a BG monitor can be contained within the remote control unit or within the dispensing unit (not shown). The patch unit 1010 as described for example in U.S. Provisional Patent Application Serial No. 61/123,509, can be directly attached to the patient's skin 5 by adhesives or other types of securing/connection mechanisms (not shown), or can be attached to a dedicated cradle unit (not shown) that is adhered to the patient skin 5 and enables the patch unit 1010 to disconnect from and/or reconnect to the body as described, for example, in co-owned, copending International Patent Application No. PCT/IL07/001578 (published as WO 2008/078318) and U.S. Patent Application No. 12/004,837 (published as US2008/0215035), claiming priority to Provisional Patent Application Serial No. 60/876,679.

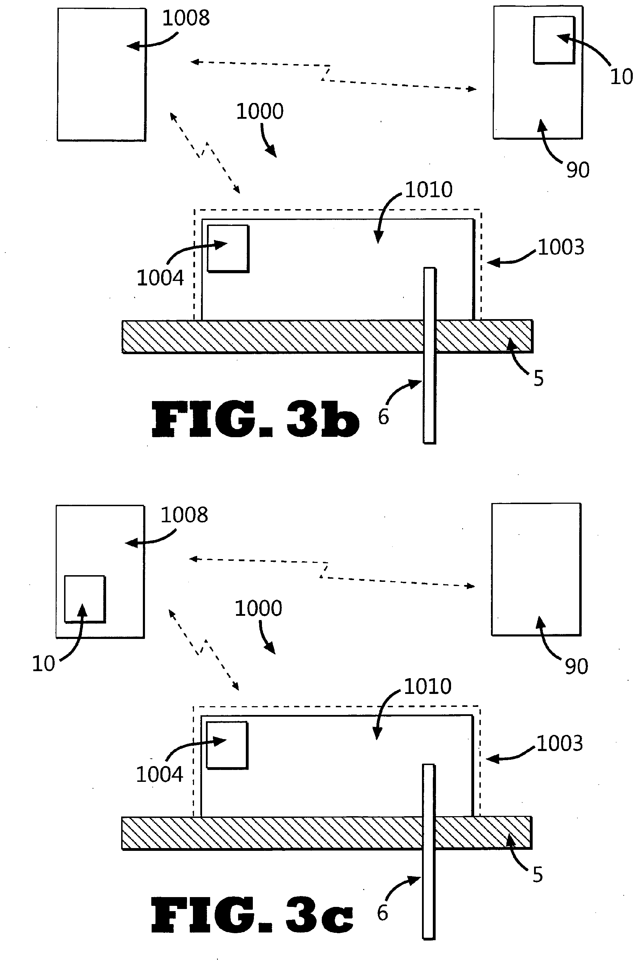

A HbAlc computation implementation 10 may be located in the sensing and dispensing unit 1010 (as shown, for example, in Figure 3a), the BG monitor 90 (shown, for example, in Figure 3b) or in the remote control unit 1008 (shown in Figures 3c-d).

In some embodiments, the system does not include a glucometer for calibration of the CGM.

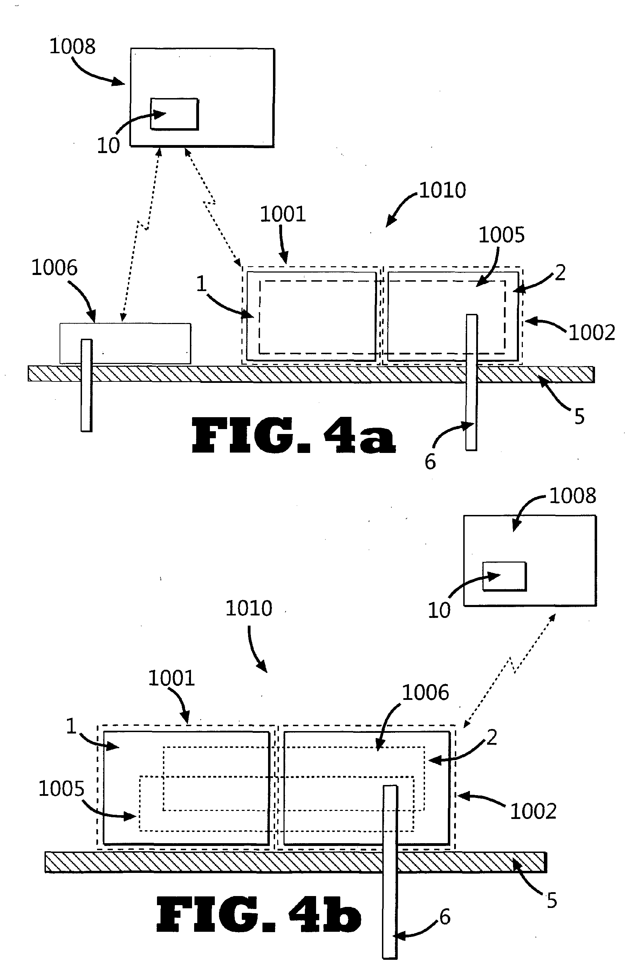

Referring to Figures 4a-b, schematic diagrams of an exemplary system 1000 that comprises a two-part patch unit that includes an insulin dispensing patch unit 1010, a remote control unit 1008 and a continuous glucose monitor (CGM) apparatus 1006 are shown. The two part patch unit includes a dispensing apparatus 1005 having a reusable part 1 and a disposable part 2. As shown, the reusable part 1 is contained in a first housing 1001 and the disposable part 2 is contained in a second housing 1002. The HbAlc computation implementation 10 can be contained within the remote control unit 1008 or within the patch unit (not shown). Figure 4a depicts a stand-alone configuration of the CGM apparatus in which continuous glucose readings can be transmitted to the remote control and patch units (indicated by the arrows). Figure 4b shows the CGM apparatus 1006 contained within a two part patch unit, which includes a portion contained within the reusable part 1 and a another portion contained within the disposable part 2. The dispensing apparatus 1005 can be connected to a cannula and the CGM apparatus 1006 can be connected to a separate probe (not shown). Alternatively, both apparatuses can be connected to a single cannula/probe as described, for example, in co-owned US Application serial No. 11/706,606 and 11/963,481, and International Patent Application No. PCT/IL07/001579, published as WO 2008/078319. In some embodiments, therapeutic fluid (e.g., insulin) can be dispensed based on, at least in part, CGM readings (i.e., in a closed loop system). In some embodiments, therapeutic fluid can be dispensed according to CGM readings and additional pre-meal bolus inputs (e.g., a semi closed loop system).

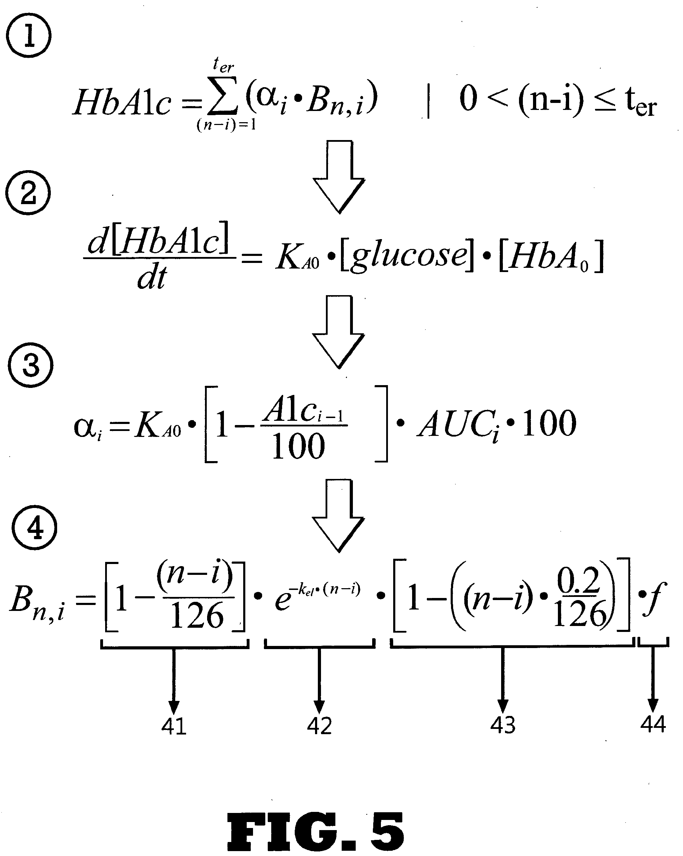

Referring to Figure 5, an exemplary mathematical representation of the model of the relationship between HbAlc and blood glucose concentrations is shown. The concentration of HbAlc in the blood is determined by the rate of its formation and removal. As described by Siv M. Ostermann-Golker and Hubert W. Vesper (Journal of Diabetes and its Complications 2006, 20,285-294), HbAlc measured at a certain day $n$ can be expressed as the sum of contributions from each of the preceding days $i$ of the 126-day erythrocyte lifespan (ter), as represented in the Equation 1, where $\alpha_i$ is the incremental increase in adduct formed at day $i$ and $B_{n,i}$ is a factor that accounts for detraction of this increment. The "adduct" refers to the formation of new HbAlc (which depends primarily on the amount of blood glucose and the amount of unreacted hemoglobin), and the "detraction" refers to a reduction in the level of HbAlc (due, for example, to the erythrocyte turnover and the HbAlc chemical stability).

The rate of formation of HbAlc may be determined in accordance with Equation 2 (shown in Figure 5), where $K_{A0}$ is the reaction rate. Equation 3 represents the percent ratio of the adduct increment $HbA1c_i$, formed during day $i$, to HbAlc, denoted as $\alpha_i$. $AUC_i$ is the area under the curve, or dose of glucose, and represents the time-weighted average of glucose concentration over the period of day $i$.

The removal, or reduction, of HbAlc is expressed as factor $B_{n,i}$, represented, for example, by Equation 4. The first factor provided in Equation 4, marked as reference numeral 41, accounts for the elimination of HbAlc due to erythrocyte turnover (erythrocyte lifespan - 126 days). The second factor of the equation, marked as reference numeral 42, accounts for the loss of HbA1c due to chemical instability, where $K_{el}$ is the reaction rate for this type of elimination. The third factor represented in Equation 4, marked as reference numeral 43, accounts for the loss of HbAlc due to spleen facilitated clearance. Spleen facilitated clearance corresponds to an additional path of erythrocytes reduction (or removal). Approximately 20% of the hemoglobin content is lost from the circulating red blood cells due to spleen-facilitated vesiculation, which is most pronounced in old cells. The fourth factor of the equation, noted as 44, accounts for the loss of HbAlc due to other types of elimination. This factor is normally 1, but, under certain circumstances that alter the RBC count (e.g., smoking, high altitude, etc.), the value of this factor may vary.

Based on clinical investigations, examples for the pharmacokinetic parameters $K_{A0}$, $K_{el}$ are given below:

$K_{A0}$ (1 mmol$^{-1}$ h$^{-1}$) = 5*10$^{-6}$ (as determined by Higgins and Bunn, 1981)

$K_{A0}$ (1 mmol$^{-1}$ h$^{-1}$) = 7.75*10$^{-6}$ (as determined by Mortensen and Volund, 1984)

$K_{el}$ (d$^{-1}$) = 0.01 (as determined by Bunn et al., 1976)

$K_{el}$ (d$^{-1}$) = 0.0045 (as determined by Saunders, 1998)

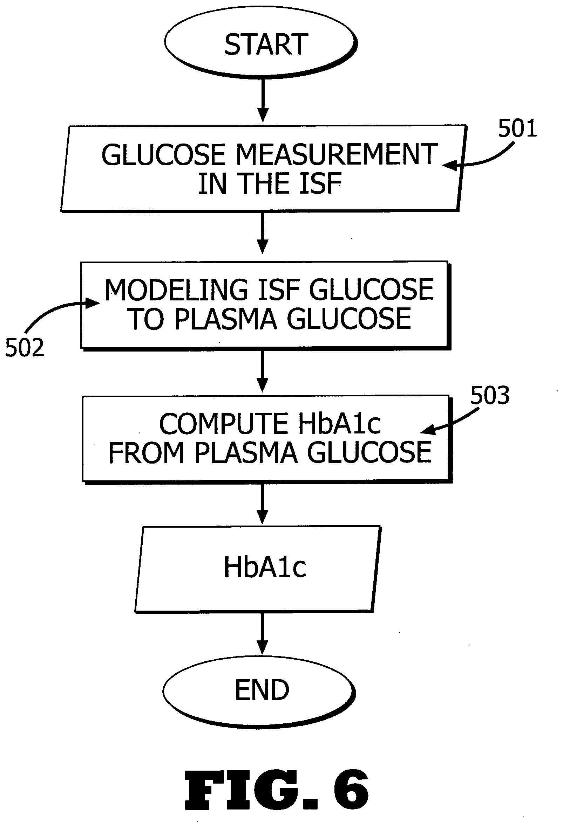

Referring to Figure 6, a schematic block diagram of an exemplary modeling (or procedural) approach to determine HbAlc level is shown.

Generally, conventional mathematical models representative of the relationship between HbAlc and bodily glucose concentrations require parameters that are determined from blood samples (e.g., plasma glucose). Consequently, a procedure to determine HbAlc levels based on such conventional model requires inconvenient blood sampling. In contrast, the system disclosed herein is attached to the patient's skin and has accessibility to the subcutaneous tissue layer through the cannula or through some other probe (e.g., nom-tubular type probe). Thus, glucose concentration levels in the ISF ("interstitial fluid") may be determined to thus enable subsequent determination of the HbAlc levels. Accordingly, and as shown in FIG. 6, the glucose concentration levels in the ISF are measured 501.

Having measured the glucose concentration levels, the measured ISF glucose levels are transformed 502 into conventional plasma glucose levels using, for example, a "transitional model". The modeled plasma glucose is then applied in a model that determines (e.g., computes) 503 the HbAlc from plasma glucose. This "integrated" modeling depicted in Figure 6 thus enables HbAlc computations using data/parameters which may be acquired using sensing/probing modules or devices disposed, for example, in the adherable patch unit, or disposed in dedicated sensing devices.

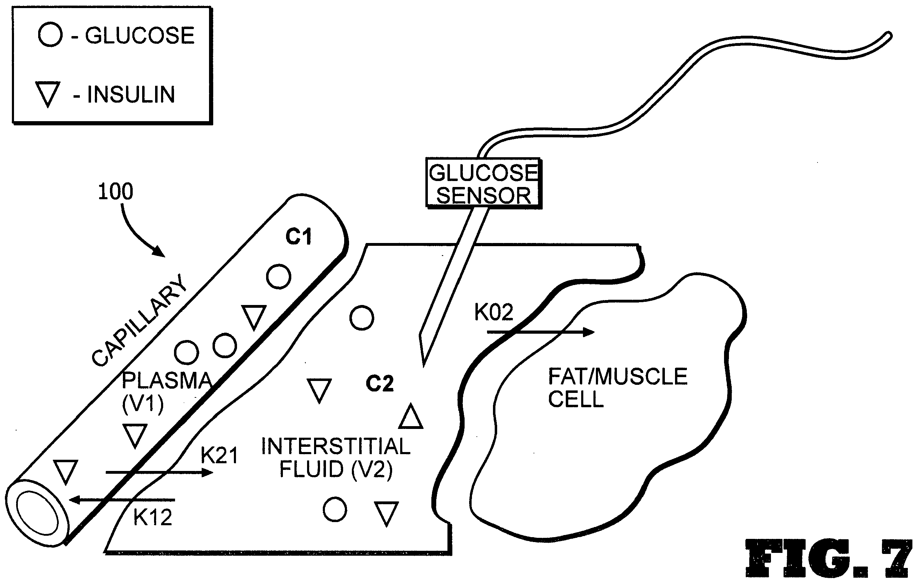

Referring to Figure 7, an anatomical/physiological schematic depicting an exemplary data acquisition model 100 to determine the glucose concentration levels corresponding to the plasma (based on which the HbAlc levels may be determined) is shown. The exemplary model 100, where subcutaneous glucose is used to estimate plasma glucose, was proposed by K. Rebrin *et al.* As shown, the model 100 describes plasma (Ci) and interstitial fluid (ISF; $C_2$) glucose kinetics. In this model it is assumed that ISF glucose equilibrates with plasma glucose by diffusion (D = $k_{21}V_1$ = $k_{12}V_2$) and that ISF glucose is cleared from ISF by tissue surrounding the sensor (clearance = $k_{02}V_2$). In this model, Vi and $V_2$ represent plasma volume and the ISF distribution volume as seen by the subcutaneously inserted sensor, respectively. To estimate the gradient and delay, the mass balance relationship for the ISF pool may be expressed as:

$$\frac{dC_2}{dt} = -\left(k_{02} + k_{12}\right)C_2 + k_{12}\frac{V_1}{V_2}C_1$$

where $C_1$ and $C_2$ are the plasma and ISF glucose concentrations, respectively. The ISF-to-plasma glucose gradient and the ISF equilibration time constant (delay) are therefore determined according to:

$$C_1 = \frac{k_{12} + k_{02}}{k_{21}\frac{V_1}{V_2}}C_2 \quad ; \quad \tau = \frac{1}{k_{12} + k_{02}}$$

(as described by K. Rebrin et al., Am J Physiol Endocrinol Metab 277:561-571, 1999)

The abovementioned modeling of plasma glucose from ISF glucose can be used to model, and thus determine, HbAlc levels from plasma glucose using a glucose sensor located in the subcutaneous tissue that measures glucose in the ISF.

In some embodiments, a model that directly derives the HbAlc value from sampled subcutaneous glucose measurements may be applied, such as the simple linear model described by Nathan D et al. (Diabetes Care 31(8),1473-1478*). For example, the regression equation for HbAlc and average glucose (AG) using a CGM is determined according to the relationship: AG = 28*HbA1c- 36.9.

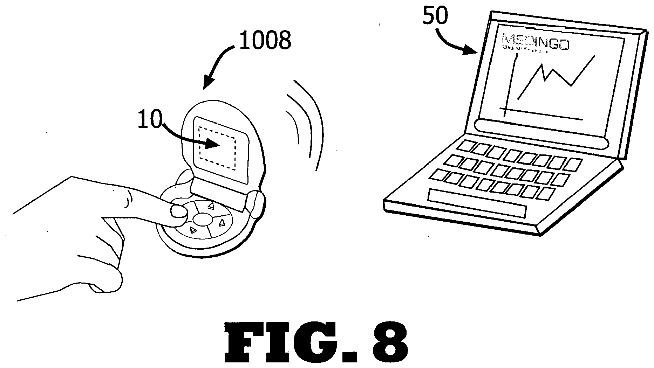

Referring to Figure 8, a diagram of an exemplary embodiment of an HbAlc computation implementation using a remote control unit 1008 and an external PC 50 is shown. Determined HbA1c level values are stored and may be displayed in any graphical or non-graphical manner. In some embodiments, the saved data may automatically be sent (e.g., by electronic mail) to the patient's practitioner for evaluation.

Any and all patents, patent applications, articles and other published and non-published documents referred to anywhere in the subject disclosure are herein incorporated by reference in their entirety.

A number of embodiments of the invention have been described. Nevertheless, it will be understood that various modifications may be made without departing from the scope of the invention, which is defined by the following claims.

**Claims**

1. A portable insulin dispensing system (1000) comprising:

   a portable insulin dispensing pump (1005) to deliver insulin to a patient;
   a glucose sensor (1006) to periodically perform a plurality of glucose measurements corresponding to glucose concentration in the blood;
   a processor (10) to determine one or more HbAlc values representative of a patient's glycosylated hemoglobin levels based on the periodic glucose measurements;
   **characterized in that**: the processor (10) is provided in any one or more of the portable insulin dispensing system (1000), a handheld remote control unit (1008) for the insulin dispensing pump and a handheld blood glucose monitor (90); the processor (10) is configured to determine one or more HbAlc values based on a mathematical model relating the one or more HbAlc values to glucose concentrations determined from the glucose measurements, with the mathematical model comprising a first order reaction kinetics model representative of a first order reaction kinetics between glucose and hemoglobin and loss of HbAlc through erythrocyte clearance in which HbAlc is estimated to be a stable reaction product; and the system is configured to alert the patient if a high HbAlc value is assessed, recommending a medical check by a health practitioner.

2. The portable insulin dispensing system (1000) of claim 1, wherein the glucose sensor (1006) is coupled to the dispensing pump (1005) and configured to periodically perform a plurality of glucose measurements in interstitial fluid.

3. The system (1000) of claim 1, wherein the system is further configured to perform setting changes to the dispensing pump (1005) if a high HbAlc value is assessed.

4. The system (1000) of claim 1, wherein the processor (10) is further configured to compute one or more values representative of the patient's glucose variability levels.

5. The system (1000) of claim 1, wherein at least part of the pump (1005) is securable to the patient's skin.

6. The system (1000) of claim 1, wherein the pump (1005) comprises the glucose sensor (1006).

7. The system (1000) of claim 1, wherein the pump comprises a disposable part (2) and a reusable part (1).

8. The system (1000) of claim 1, wherein the one or more HbAlc values are computed based on a time-weighted averaged computation of the glucose measurements.

**Patentansprüche**

1. Tragbares Insulinabgabesystem (1000), umfassend:

   eine tragbare Insulinabgabepumpe (1005) zur Abgabe von Insulin an einen Patienten,
   einen Glukosesensor (1006), um periodisch eine Vielzahl von Glukosemessungen vorzunehmen, die der Glukosekonzentration im Blut entsprechen,
   einen Prozessor (10) zur Bestimmung eines oder mehrerer HbAlc-Werte, die für die glykosylierten Hämoglobinwerte eines Patienten repräsentativ sind, basierend auf den periodischen Glukosemessungen,
   **dadurch gekennzeichnet, dass**
   der Prozessor (10) in einem oder mehreren der tragbaren Insulinabgabesysteme (1000), einer Handfernbedienung (1008) für die Insulinabgabepumpe und einem tragbaren Blutzuckermessgerät (90) vorgesehen ist,
   der Prozessor (10) ausgebildet ist, um einen oder mehrere HbAlc-Werte auf der Grundlage eines mathematischen Modells zu bestimmen, das den einen oder die mehreren HbAlc-Werte mit aus den Glukosemessungen bestimmten Glukosekonzentrationen in Beziehung setzt, wobei das mathematische Modell ein Reaktionskinetikmodell erster Ordnung umfasst, das für eine Reaktionskinetik erster Ordnung zwischen Glukose und Hämoglobin und den Verlust von HbAlc durch Erythrozyten-Clearance steht, wobei HbAlc als ein stabiles Reaktionsprodukt eingeschätzt wird, und

das System so ausgebildet ist, dass es den Patienten alarmiert, wenn ein überhöhter HbAlc-Wert festgestellt wird, und eine ärztliche Untersuchung durch einen Mediziner empfiehlt.

2. Tragbares Insulinabgabesystem (1000) nach Anspruch 1, wobei der Glukosesensor (1006) mit der Abgabepumpe (1005) gekoppelt und so ausgebildet ist, dass er periodisch eine Vielzahl von Glukosemessungen in Zwischenzellflüssigkeit vornimmt.

3. System (1000) nach Anspruch 1, wobei das System ferner so ausgebildet ist, dass es Einstellungsänderungen an der Dosierpumpe (1005) durchführt, wenn ein überhöhter HbAlc-Wert festgestellt wird.

4. System (1000) nach Anspruch 1, wobei der Prozessor (10) ferner so ausgebildet ist, dass er einen oder mehrere Werte berechnet, die für die Glukosevariabilitätsstufen des Patienten repräsentativ sind.

5. System (1000) nach Anspruch 1, wobei mindestens ein Teil der Pumpe (1005) an der Haut des Patienten befestigbar ist.

6. System (1000) nach Anspruch 1, wobei die Pumpe (1005) den Glukosesensor (1006) umfasst.

7. System (1000) nach Anspruch 1, wobei die Pumpe einen Einwegteil (2) und einen wiederverwendbaren Teil (1) umfasst.

8. System (1000) nach Anspruch 1, wobei der eine oder die mehreren HbA1c-Werte auf der Grundlage einer zeitgewichteten gemittelten Berechnung der Glukosemessungen berechnet werden.

**Revendications**

1. Système d'administration d'insuline portable (1000) comprenant :

une pompe d'administration d'insuline portable (1005) pour administrer de l'insuline à un patient ;

un détecteur de glucose (1006) pour effectuer périodiquement une pluralité de mesures de glucose correspondant à la concentration de glucose dans le sang ;

un processeur (10) pour déterminer une ou plusieurs valeurs d'HbA1c représentatives des niveaux d'hémoglobine glycosylée d'un patient sur la base des mesures périodiques du glucose ;

**caractérisé en ce que** :

le processeur (10) est fourni dans l'un ou plusieurs du système d'administration d'insuline portable (1000), d'un module de commande à distance portatif (1008) pour la pompe d'administration d'insuline et d'un contrôleur de glycémie portatif (90) ; le processeur (10) est configuré pour déterminer une ou plusieurs valeurs d'HbA1c sur la base d'un modèle mathématique rapportant la ou les valeurs d'HbA1c aux concentrations de glucose déterminées d'après les mesures de glucose, le modèle mathématique comprenant un modèle cinétique de réaction de premier ordre représentatif d'une cinétique de réaction de premier ordre entre le glucose et l'hémoglobine et la perte d'HbA1c par clairance des érythrocytes, l'HbAlc étant estimé être un produit de réaction stable ; et le système est configuré pour alerter le patient si une valeur d'HbA1c élevée est estimée, en recommandant un contrôle médical par un professionnel de la santé.

2. Système d'administration d'insuline portable (1000) selon la revendication 1, dans lequel le détecteur de glucose (1006) est couplé à la pompe d'administration (1005) et configuré pour effectuer périodiquement une pluralité de mesures du glucose dans le fluide interstitiel.

3. Système (1000) selon la revendication 1, le système étant en outre configuré pour effectuer des changements de réglage de la pompe d'administration (1005) si une valeur d'HbA1c élevée est estimée.

4. Système (1000) selon la revendication 1, dans lequel le processeur (10) est en outre configuré pour calculer une ou plusieurs valeurs représentatives des niveaux de variabilité du glucose du patient.

5. Système (1000) selon la revendication 1, dans lequel au moins une partie de la pompe (1005) peut être fixée à la peau du patient.

6. Système (1000) selon la revendication 1, dans lequel la pompe (1005) comprend le détecteur de glucose (1006).

7. Système (1000) selon la revendication 1, dans lequel la pompe comprend une partie jetable (2) et une partie réutilisable (1).

8.  Système (1000) selon la revendication 1, dans lequel la ou les valeurs d'HbAlc sont calculées sur la base d'un calcul moyen pondéré dans le temps des mesures de glucose.

| HbA1c (%) | AVG. BLOOD SUGAR (mmol/L) | AVG. BLOOD SUGAR (mg/dL) |
|---|---|---|
| 5 | 4.5 | 80 |
| 6 | 6.7 | 120 |
| 7 | 8.3 | 150 |
| 8 | 10.0 | 180 |
| 9 | 11.6 | 210 |
| 10 | 13.3 | 240 |
| 11 | 15.0 | 270 |
| 12 | 16.7 | 300 |

# FIG. 1

1008

1000

10

1020

1003

5

6

# FIG. 2a

1008

10

1000

1020

1003

5

6

# FIG. 2b

**FIG. 2c**

**FIG. 3a**

# FIG. 3b

# FIG. 3c

## FIG. 3d

**FIG. 4a**

**FIG. 4b**

① 
$$HbA1c = \sum_{(n-i)=1}^{t_{er}} (\alpha_i \cdot B_{n,i}) \quad | \quad 0 < (n-i) \le t_{er}$$

② 
$$\frac{d[HbA1c]}{dt} = K_{A0} \cdot [glucose] \cdot [HbA_0]$$

③ 
$$\alpha_i = K_{A0} \cdot \left[ 1 - \frac{A1c_{i-1}}{100} \right] \cdot AUC_i \cdot 100$$

④ 
$$B_{n,i} = \left[ 1 - \frac{(n-i)}{126} \right] \cdot e^{-k_{el} \cdot (n-i)} \cdot \left[ 1 - \left( (n-i) \cdot \frac{0.2}{126} \right) \right] \cdot f$$

41     42     43     44

# FIG. 5

FIG. 6

FIG. 2

# FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61009296 A
- US 3771694 A
- US 4657486 A
- US 4498843 A
- US 5957895 A, Burton
- US 6589229 B, Connelly
- US 6740059 B, Flaherty
- US 6723072 B
- US 6485461 B
- US 397115 A
- US 20070106218 A
- US 6558351 B
- US 706606 A
- US 11963481 B
- IL 07001579 W
- WO 2008078319 A
- EP 1845465 A
- US 20040096959 A
- US 61123509 A
- IL 07001578 W
- WO 2008078318 A
- US 004837 A
- US 20080215035 A
- WO 60876679 A

### Non-patent literature cited in the description

- DCCT Trial. *N Engl J Med,* 1993, vol. 329, 977-986
- UKPDS Trial. *Lancet,* 1998, vol. 352, 837-853
- *BMJ,* 1998, vol. 317 (7160), 703-13
- EDIC Trial. *N Engl J Med,* 2005, vol. 353 (25), 2643-53
- **SIV M. OSTERMANN-GOLKER ; HUBERT W. VESPER.** *Journal of Diabetes and its Complications,* 2006, vol. 20, 285-294
- *Diabetes Care,* 2006, vol. 29 (2), 466-467
- *Diabetes,* 2004, vol. 53, 955-962
- **K. REBRIN et al.** *Am J Physiol Endocrinol Metab,* 1999, vol. 277, 561-571
- **NATHAN D et al.** *Diabetes Care,* vol. 31 (8), 1473-1478